# EUROPEAN PATENT SPECIFICATION

(11) **EP 0 616 847 B1**
(45) Date of publication and mention of the grant of the patent: **19.11.1997**
(21) Application number: 94104704.5
(22) Date of filing: 24.03.1994
(51) Int. Cl.: B01J 27/185, B01J 27/188, C10G 45/08

(54) **Solid acid catalyst for paraffin conversion and process for paraffin conversion using the same**
Fester Säurenkatalysator für die Umwandlung von Paraffinen und Verfahren für die Paraffinumwandlung unter Verwendung derselben
Catalyseur solide acide pour la conversion des paraffines et procédé de conversion des paraffines l'utilisant

(30) Priority: 26.03.1993 JP 92125/93
(43) Date of publication of application: 28.09.1994
(73) Proprietor: NIPPON OIL CO. LTD., Minato-ku Tokyo (JP)
(72) Inventor: Na, Kyutae, A-126, Tokyo-daigaku Mitaka, Mitaka-shi, Tokyo-to 181 (JP); Okuhara, Toshio, 1-201, Tokyo-daigaku Nishi, Chiba-shi, Chiba-ken, 263 (JP); Misono, Makoto, Tokyo, 166 (JP)
(74) Representative: Hansen, Bernd, Dr. Dipl.-Chem.

(56) References cited:
- EP-A- 0 353 722
- EP-A- 0 363 202
- EP-A- 0 382 644
- EP-A- 0 466 471
- EP-A- 0 494 528

## Description

### BACKGROUND OF THE INVENTION

### 1) Field of the Invention

The present invention relates to a solid acid catalyst for paraffin conversion which is used in petroleum refining and petrochemical industries and a process for paraffin conversion using catalyst thereof.

### 2) Related Prior Art

In petroleum refining and petrochemical industries, conversion reactions participating in a large number of paraffins have been carried out. The example includes catalytic cracking, catalytic reforming, hydrocracking, isomerization, alkylation, etc.

Particularly, as a catalyst for conversion carrying out paraffin isomerization reaction and paraffin alkylation reaction, etc., an acid catalyst having strong acidity is required. Hitherto, for these reactions, acid catalysts such as sulfuric acid, hydrogen fluoride, aluminum chloride and antimony trichloride have been used. However, since these catalysts cause metal corrosion, use of expensive materials having corrosion resistance and treatment for anti-corrosion are required. Moreover, with recent tightening of environmental regulations, disposal of waste catalysts requires a high cost and tends to severely be regulated. Furthermore, there has been the problem that these catalysts are difficult to separate from the reactants.

Moreover, in paraffin isomerization, there has been applied a process which comprises carrying out isomerization in the presence of a catalyst treated a solid acid supported a noble metal including Pt with a compound containing a halogen, and hydrogen. However, since the catalyst is very readily deactivated with water, there has been the problem that a higher cost is required for removal of water in a starting material.

JP-A-5-58921 discloses a process for producing a butyl group-substituted aromatic compound which comprises reacting ethylene with an aromatic compound in the presence of a catalyst containing an alkali metal salt of heteropoly acid and a metal of Group VIII. However, JP-A-5-58921 disclose no paraffin conversion.

EP-A-0 363 202 and EP-A-0 466 471 disclose alkane oxidation which occurs in the presence of a catalyst, the catalyst being a heteropoly acid comprising a group VIII metal.

### SUMMARY OF THE INVENTION

The object of the present invention is to solve the above-mentioned prior problems and provide a solid acid catalyst for paraffin conversion which causes no corrosion and is readily separated from reactants and furthermore has high activity for an acid catalyst reaction.

As a result of an extensive study to solve the above-mentioned problems, the present inventors have found that a catalyst comprising specific components, particularly a catalyst comprising a specific composition of components causes no corrosion and is readily separated from reactants and furthermore has high activity for a solid acid catalyst reaction for paraffin conversion, and has established the present invention.

The present invention provides a solid acid catalyst for paraffin conversion as described in claim 1.

The present invention provides also a process for paraffin conversion which comprises carrying out paraffin conversion in the presence of a solid acid catalyst as described in claim 7.

Moreover, the present invention provides a process for paraffin isomerization which comprises carrying out paraffin isomerization in the presence of a solid acid catalyst as described in claim 13.

### DETAILED DESCRIPTION OF THE INVENTION

The present invention will be described in detail below.

The term which is a components of the catalyst according to the present invention " acidic salt of heteropoly acid " means a substance wherein a portion of hydrogen ions in a heteropoly acid has been replaced by other cations.

The term " heteropoly acid " means a general term of the acid wherein two or more species of inorganic oxyacids have been condensed. Although a heteropoly acid for use in the present invention is not limited to a specific structure, the heteropoly acid represented by the general formula (1) is preferable.

H₍₈₀₋ₘ₋₁₂ₙ₎[X^{m+}Yⁿ⁺₁₂O₄₀] (1)

wherein X is called hetero-atom, which is one atom selected from the group consisting of P, As, Si and Ge ; Y is called poly-atom, which is at least one transition metal from the group consisting of Mo, W, Nb and V ; m and n represent a valence number of X and Y, respectively.

Among the heteropoly acids represented by the general formula (1), the heteropoly acid is preferable wherein hetero-atom is P or Si and the heteropoly acid is preferable wherein poly-atom is W or Mo. H₃PW₁₂O₄₀ and H₃PMₒ₁₂O₄₀ are more preferable. Particularly, H₃PW₁₂O₄₀ is most preferable.

The catalyst for use in the present invention comprises an acidic salt of the heteropoly acid in which a portion of hydrogen ions are replaced by the other cations and a metal of Group VIII. The replacing cations are alkali metal ion, alkaline earth metal ion and ammonium ion. Among them, K⁺, Rb⁺, Cs⁺ and NH₄⁺ are more preferable. Particularly, Cs⁺ is most preferable.

In preparation of the catalyst, a salt of the cations reacts with a heteropoly acid. As the salts for use in the preparation, carbonates, nitrates, sulfates, chlorides, acetates and oxalates are preferable. Particularly, carbonates are more preferable.

The salt acid of heteropoly acid for use in the present invention is preferably represented by the following general formula (2):

L^{l+}ₖH_{(80-m-12n-k·l)}[X^{m+}Yⁿ⁺₁₂O₄₀] (2)

wherein L is one ion selected from the group consisting of alkali metal ion, alkaline earth metal and ammonium ion ; X represents a hetero-atom ; Y represents a polyatom ; ℓ, m and n represent a valence number of L, X and Y, respectively ; k represents the number of L and a number of over 0 and below (80-m-12n)/ℓ.

The component of acid salt of heteropoly acid for use in the present invention is more preferably represented by the following general formula (3) wherein the hetero-atom is P and the poly-atom is at least one atom selected the group consisting of W and Mo:

AₓH₍₃₋ₓ₎PY₁₂O₄₀ (3)

wherein A represents one ion selected from the group consisting of alkali metal ion, and ammonium ion ; Y represents at least one atom selected from the group consisting of W and Mo ; X represents the number of A.

X in the acid salt of heteropoly acid for use in the present invention is preferably 2.05 to 2.95, more preferably 2.1 to 2.9, most preferably 2.2 to 2.8. When X is below 2.05, activity is low and hydrocracking which is a generally unfavorable side reaction predominatly occurs. Moreover, when X exceeds 2.95, hydrocracking which is a generally unfavorable side reaction predominatly occurs.

The metal of Group VIII for use in the present invention is one metal belonging to Group VIII of the Periodic Table. Among the metals, Ru, Rh, Pd, Os, Ir or Pt is preferable. Particularly, Pd or Pt is more preferable. As the salts employing in preparation of the catalyst, optional salts which are ordinarily available may be employed. For example, halogenides, nitrates, sulfates, acetates, cyanides, acetylacetonates, ammine ammine complexes and carbonyl compylexes and the like are preferable. Preferable example includes, platinum chloride, platinum iodide, chloroplatinic acid, potassium chloroplatinate, Pt(NH₃)₄Cl₂, palladium acetate, palladium chloride, palladium nitrate, palladium acetylacetnate, rhodium acetate, rhodium chloride, rhodium nitrate, ruthenium chloride, osmium chloride and iridium chloride.

The content of a metal of Group VIII in the present invention is 0.05 to 30% by weight, preferably 0.2 to 10% by weight, most preferably 0.5 to 5% by weight, based on the total amount of a catalyst.

The process for preparation of the catalyst comprising an acidic salt of a heteropoly acid and a metal of Group VIII in the present invention is not particularly limited. In the process it is preferable that a metal salt of Group VIII contacts with a heteropoly acid and subsequently is neutralized with an alkali metal salt or ammonium salt to produce an acidic salt. When a metal of Group VIII is supported after an acidic salt of a heteropoly acid has been prepared, activity tends to deteriorate.

The catalyst according to the present invention may be used alone or supported on a carrier. As the carrier, optional carriers may be used. The example includes metal oxides such as silica, alumina, etc., metal composite oxides such as silica·alumina, etc., zeolite and active carbon. Among them, silica, alumina, titania, zirconia and active carbon are particularly more preferable.

It is desirable that pretreatment is carried out in an oxidation atmosphere for the catalyst according to the present invention. Example of the pretreatment in an oxidation atmosphere includes treatment in oxygen gas and treatment in air. By carrying out the pretreatment in an oxidation atmosphere, activity and selectivity including paraffin isomerization reaction further increase. Before or after the pretreatment in an oxidation atmosphere, treatment in nitrogen gas or an inert gas may be carried out. It is desirable that pretreament in a reduction atmosphere including treatment in hydrogen is carried out before pretreatment in an oxidation atmosphere. When pretreatment in a reduction atmosphere is carried out after pretreatment in an oxidation atmosphere, significant advantages being provided by the pretreatment in an oxidation atmosphere tend to decrease.

The catalyst according to the present invention is effective in paraffin conversion reactions . The example includes catalytic cracking, catalytic reforming, hydrocracking, isomerization, alkylation, etc. Particularly, the catalyst according to the present invention is effective in paraffin isomerization reactions and paraffin alkylation reactions.

As paraffins for use in the present invention, a paraffin having 4 to 12 carbon atoms is preferable. Particularly, a paraffin having 4 to 8 carbon atoms is more preferable. Preferable example for use includes butane, pentane, hexane, heptane and octane. The paraffin can be used alone or in a mixture of paraffins. Moreover, a light naphtha containing 50% by weight or above of paraffins also can be used. Particularly, butane, pentane and hexane are more preferable.

Paraffin isomerization reaction is described in details below. Paraffin isomerization reaction can be carried out either batchwise or by one-path flow. Batchwise, the amount of the catalyst is preferably 0.1 to 10% by weight, more preferably 1 to 5% by weight, based on a starting material.

The reaction temperature is preferably 70 to 400°C, more preferably 100 to 350°C. The reaction pressure may be in atmospheric pressure or an applied pressure. In the case of one-path flow, liquid hour space velocity (LHSV) is preferably 0.1 to 10 hr⁻¹, more preferably 0.3 to 4 hr⁻¹.

The catalytic conversion according to the present invention is carried out in the presence of hydrogen. By carrying it out in the presence of hydrogen, activity and selectivity in isomerization further increase and catalyst life tends to extend. The concentration of hydrogen in reaction gases is preferably 0.1 to 99% by volume, more preferably 0.5 to 80% by volume, most preferably 1 to 60% by volume.

### PREFERRED EMBODIMENTS OF THE INVENTION

The present invention will be described in details below, referring to Examples and Comparative Examples, which are not limitative of the present invention.

### Example 1.

10.96 g of H₃PW₁₂O₄₀ · 6H₂O was mixed with water to obtain an aqueous solution of 0.08 mol/ℓ and then a solution of 0.3024 g Pt(NH₃)₄Cl₂, dissolved in 10 ml of water was gradually dropped therein while stirring at 50°C. After leaving for 30 minutes, an aqueous caesium carbonate solution of 0.125 mol/ℓ was gradually dropped therein. After leaving at room temperature over one night, water was evaporated for drying by a rotary evaporator to obtain Pt-Cs_{2.5}H_{0.5}PW₁₂O₄₀. The catalyst obtained thus contained 1.5 wt% of Pt and had a surface area of 100 m²/g.

Butane isomerization reaction was carried out using a fixed bed-one path flow reactor. 1 g of Pt-Cs_{2.5}H_{0.5}PW₁₂O₄₀ was charged to the reactor. After pretreatment had been carried out at 300°C for one hour in oxygen gas and for one hour in nitrogen gas, reaction was carried out at a reaction temperature of 300°C under 0.1 MPa (1 atm) while flowing a mixed gas of butane 5% by volume, H₂ 50% by volume and N₂ 45% volume at a flow rate of 10 ml/min. Conversion rate at 5 hours after starting of reaction, selectivity to isobutane and production rate of isobutane are shown in table 1. Products excluding isobutane of isomerization reaction product were mostly methane, ethane and propane.

### Example 2

Butane isomerization reaction was carried out in the same manner as in Example 1 except that a reaction gas of butane 5% by volume, H₂ 5% by volume, N₂ 45% by volume and He 45% by volume was used.

### Example 3

10.86 g of H₃PW₁₂O₄₀ · 6H₂O was mixed with water to obtain an aqueous solution of 0.08 mol/ℓ and then a solution of 0.4233 g Pd(NO₃)₂ · 1.5 H₂O dissolved in 5 ml of water was gradually dropped therein while stirring at 50°C. After leaving for 30 minutes, an aqueous caesium carbonate solution of 0.125 mol/ℓ was gradually dropped therein. After leaving at room temperature over one night, water was evaporated for drying by a rotary evaporator to obtain Pd-Cs_{2.5}H_{0.5}PW₁₂O₄₀. The catalyst obtained thus contained 1.5 wt% of Pd and had a surface area of 113 m²/g.

Butane isomerization reaction was carried out in the same manner as in Example 1 except that 1 g of Pd-Cs_{2.5}H_{0.5}PW₁₂O₄₀ was used in place of 1 g of Pt-Cs_{2.5}H_{0.5}PW₁₂O₄₀. The result is shown in table 1.

### Example 4.

Butane isomerization reaction was carried out in the same manner as in Example 3 except that a reaction gas of butane 5% by volume, H₂ 5% by volume, N₂ 45% by volume and He 45% by volume. The result is shown in table 1.

### Comparative Example 1.

8.562 g of H₃PW₁₂O₄₀ · 6H₂O was mixed with water to obtain an aqueous solution of 0.08 mol/ℓ and then a solution of 0.2118 g Pt(NH₃)₄Cl₂ dissolved in 5 ml of water was gradually dropped therein while stirring at 50°C. After leaving at room temperature over one night, water was evaporated for drying by a rotary evaporator to obtain Pt-H₃PW₁₂O₄₀. The catalyst obtained thus contained 1.5 wt% of Pt and had a surface area of 9.7 m²/g.

Butane isomerization reaction was carried out in the same manner as in Example 1 except that 1 g of Pt-H₃PW₁₂O₄₀ was used in place of 1 g of Pt-Cs_{2.5}H_{0.5}PW₁₂O₄₀. The result is shown in table 1.

### Comparative Example 2.

8.203 g of H₃PW₁₂O₄₀ · 6H₂O was mixed with water to obtain an aqueous solution of 0.08 mol/ℓ and then a solution of 0.2866 g Pd(NO₃)₂ · 1.5H₂O dissolved in 5 ml of water was gradually dropped therein while stirring at 50°C. After leaving at room temperature over one night, water was evaporated for drying by a rotary evaporator to obtain Pd-H₃PW₁₂O₄₀. The catalyst obtained thus contained 1.5 wt% of Pd and had a surface area of 8.6 m²/g.

Butane isomerization reaction was carried out in the same manner as in Example 1 except that 1 g of Pd-H₃PW₁₂O₄₀ was used in place of 1 g of Pt-Cs_{2.5}H_{0.5}PW₁₂O₄₀. The result is shown in table 1.

### Comparative Example 3

Butane isomerization reaction was carried out in the same manner as in Example 1 except that 0.5 g of catalyst supported 1 wt% of Pt on H-ZSM-5 (25H, produced by Mobil Catalysts Corporation of Japan) was used in place of Pt-Cs_{2.5}H_{0.5}PW₁₂O₄₀. The result is shown in table 1.

### Comparative Example 4

Butane isomerization reaction was carried out in the same manner as in Example 1 except that 1 wt% of Pt/Al₂O₃ (produced by N.E.Chemcat Co.) was used in place of Pt-Cs_{2.5}H_{0.5}PW₁₂O₄₀.

### Comparative Example 5

Pt-Cs₃PW₁₂O₄₀ was prepared in the same manner as in Example 1 except that the dropping amount of the aqueous caesium carbonate solution was changed and as the pretreatment of catalyst, oxygen gas treatment was carried out after nitrogen gas treatment.

Butane isomerization reaction was carried out in the same manner as in Example 1 except that 1 g of Pt-Cs₃PW₁₂ O₄₀ was used in place of 1 g of Pt-Cs_{2.5}H_{0.5}PW₁₂ O₄₀. The result is shown in table 1.

### Comparative Example 6

Butane isomerization reaction was carried out in the same manner as in Example 1 except that as reaction gases, butane 5% by volume and N₂ 95% by volume were used. The result is shown in table 1.

### Comparative Example 7

Butane isomerization reaction was carried out in the same manner as Example 3 except that as reaction gases, butane 5% by volume and N₂ 95% by volume were used. The result is shown in table 1.

### Comparative Example 8

H₃PW₁₂O₄₀·6H₂O was mixed with water to obtain an aqueous solution of 0.08 mol/ℓ and then an aqueous caesium carbonate solution of 0.125 mol/ℓ was gradually dropped therein while stirring. After leaving over one night, water was evaporated for drying by a rotary evaporator to obtain Cs_{2.5}H_{0.5}PW₁₂O₄₀.

Butane isomerization reaction was carried out in the same manner as in Example 1 except that 1 g of Cs_{2.5}H_{0.5}PW₁₂O₄₀ was used in place of 1 g of Pt-Cs_{2.5}H_{0.5}PW₁₂O₄₀. The result is shown in table 1.

### Comparative Example 9

Ammonia water was added to an aqueous solution of zirconium oxychloride to produce precipitation. After filtration and drying of the precipitation, 0.05 mol/ℓ of sulfuric acid was poured and dried. An aqueous solution of chloroplatinic acid was impregnated therein and then dried and calcined to obtain Pt-supported-sulfuric acid treated ZrO₂ catalyst (hereinafter, referred to Pt-SO₄²⁻/ZrO₂).

Butane isomerization reaction was carried out in the same manner as in Example 1 except that 1 g of Pt-SO₄²⁻/ZrO₂ was used in place of 1 g of Pt-Cs_{2.5}H_{0.5}PW₁₂O₄₀.

### Example 5

Pentane isomerization reaction was carried out using a fixed bed-high pressure-one path flow reactor. 1 g of Pt-Cs_{2.5}H_{0.5}PW₁₂O₄₀ obtained in Example 1 was charged to the reactor. After pretreatment had been carried out at 300°C for one hour in oxygen gas and for one hour in nitrogen gas, reaction was carried out at a reaction temperature of 170°C under total pressure of 2 MPa (20 kg/cm²) in H₂/pentane ratio of 3/2 at WHSV of 4h⁻¹. Conversion rate at 5 hour after starting of reaction and isopentane selectivity are shown in table 2. Products excluding isopentane of isomerization reaction product were mostly methane, ethane, propane and butane.

### Example 6

Pentane isomerization reaction was carried out in the same manner as in Example 5 except that 1 g of Pd-Cs_{2.5}H_{0.5}PW₁₂O₄₀ obtained in Example 3 was used in place of 1 g of Pt-Cs_{2.5}H_{0.5}PW₁₂O₄₀. The result is shown in table 2.

### Example 7

Pt-Cs_{2.4}H_{0.6}PW₁₂O₄₀ was prepared in the same as in Example 1 except that the dropping amount of the aqueous solution of caesium carbonate was changed.

Pentane isomerization reaction was carried out in the same manner as in Example 5 except that 1 g of Pt-Cs_{2.4}H_{0.6}PW₁₂O₄₀ was used in place of 1 g of Pt-Cs_{2.5}H_{0.5}PW₁₂O₄₀. The result is shown in table 2.

### Example 8

Pt-Cs_{2.6}H_{0.4}PW₁₂O₄₀ was prepared in the same manner as in Example 1 except that the dropping amount of the aqueous solution of caesium carbonate was changed.

Pentane isomerization reaction was carried out in the same manner as in Example 5 except that 1 g of Pt-Cs_{2.6}H_{0.4}PW₁₂O₄₀ was used in place of 1 g of Pt-Cs_{2.5}H_{0.5}PW₁₂O₄₀. The result is shown in table 2.

### Reference Example 1

Pt-CsH₂PW₁₂O₄₀ was prepared in the same manner as in Example 1 except that the dropping amount of the aqueous solution of caesium carbonate was changed.

Pentane isomerization reaction was carried out in the same manner as in Example 5 except that 1 g of Pt-CsH₂PW₁₂O₄₀ was used in place of 1 g of Pt-Cs_{2.5}H_{0.5}PW₁₂O₄₀. The result is shown in table 2.

### Reference Example 2

Pt-Cs₂HPW₁₂O₄₀ was prepared in the same manner as in Example 1 except that the dropping amount of the aqueous solution of caesium carbonate was changed.

Pentane isomerization reaction was carried out in the same manner as in Example 5 except that 1 g of Pt-Cs₂HPW₁₂O₄₀ was used in place of 1 g of Pt-Cs_{2.5}H_{0.5}PW₁₂O₄₀. The result is shown in table 2.

### Comparative Example 12

Pentane isomerization reaction was carried out in the same manner as in Example 5 except that 1 g of Pt-Cs₃PW₁₂O₄₀ obtained in Comparative Example 5 was used in place of 1 g of Pt-Cs_{2.5}H_{0.5}PW₁₂O₄₀. The result is shown in table 2.

### Comparative Example 13

Pentane isomerization reaction was carried out in the same manner as in Example 5 except that 1 g of Pt-SO₄²⁻/ZrO₂ obtained in Comparative Example 9 was used in place of 1 g of Pt-Cs_{2.5}H_{0.5}PW₁₂O₄₀. The result is shown in table 2.

As is clear from tables 1 and 2, the catalyst according to the present invention has high selectivity for isobutane and isopentane and conversion rate in the conversion reaction of butane and pentane which is a paraffin conversion reaction. Moreover, the catalyst according to present invention is non-corrosive and readily separated from reactants.

Thus, the catalyst according to the present invention is a superior catalyst having high activity for paraffin conversion reaction.

**Table 1**

| Butane isomerization reaction | | | | | |
|---|---|---|---|---|---|
| Example or Comp.Ex. | species of catalyst | H₂ concentration(a) | conversion (b) | selectivity (c) | production rate(d) |
| Example 1 | Pt-Cs_{2.5}H_{0.5}PW₁₂O₄₀ | 50 | 24.6 | 93.8 | 7.9 |
| Example 2 | Pt-Cs_{2.5}H_{0.5}PW₁₂O₄₀ | 5 | 20.5 | 88.2 | 6.2 |
| Example 3 | Pd-Cs_{2.5}H_{0.5}PW₁₂O₄₀ | 50 | 33.6 | 95.5 | 10.9 |
| Example 4 | Pd-Cs_{2.5}H_{0.5}PW₁₂O₄₀ | 5 | 12.9 | 78.3 | 3.4 |
| Comp. Ex.1 | Pt-H₃PW₁₂O₄₀ | 50 | 4.2 | 95.4 | 1.3 |
| Comp. Ex.2 | Pd-H₃PW₁₂O₄₀ | 50 | 7.3 | 86.2 | 2.1 |
| Comp. Ex.3 | Pt-H-ZSM-5 | 50 | 51.0 | 34.0 | 11.8 |
| Comp. Ex.4 | Pt-Al₂O₃ | 50 | 75.0 | 8.0 | 2.0 |
| Comp. Ex.5 | Pt-Cs₃PW₁₂O₄₀ | 50 | 31.0 | 2.5 | 0.3 |
| Comp. Ex.6 | Pt-Cs_{2.5}H_{0.5}PW₁₂O₄₀ | 0 | 2.5 | 25.9 | 0.2 |
| Comp. Ex.7 | Pd-Cs_{2.5}H_{0.5}PW₁₂O₄₀ | 0 | 2.3 | 12.5 | 0.09 |
| Comp. Ex.8 | Cs_{2.5}H_{0.5}PW₁₂O₄₀ | 50 | 5.5 | 87.3 | 1.6 |
| Comp. Ex.9 | Pt-SO₄²⁻/ZrO₂ | 50 | 65.0 | 47.3 | 10.4 |

| | | | | | |
|---|---|---|---|---|---|
| (a) H₂ concentration in reaction gases, Vol% | | | | | |
| (b) conversion of butane, mol% | | | | | |
| (c) selectivity to isobutane, mol% | | | | | |
| (d) production rate of isobutane, 10⁻⁸mol/g·s | | | | | |

**Table 2**

| Pentane isomerization reaction | | | |
|---|---|---|---|
| Example | species of catalyst | conversion (a) | selectivity (b) |
| Example 5 | Pt-Cs_{2.5}H_{0.5}PW₁₂O₄₀ | 64 | 100 |
| Example 6 | Pd-Cs_{2.5}H_{0.5}PW₁₂O₄₀ | 65 | 100 |
| Example 7 | Pt-Cs_{2.4}H_{0.6}PW₁₂O₄₀ | 60 | 98 |
| Example 8 | Pt-Cs_{2.6}H_{0.4}PW₁₂O₄₀ | 59 | 99 |
| Ref. Ex. 1 | Pt-CsH₂PW₁₂O₄₀ | 5 | 8 |
| Ref. Ex. 2 | Pt-Cs₂HPW₁₂O₄₀ | 7 | 6 |
| Comp. Ex.12 | Pt-Cs₃PW₁₂O₄₀ | 71 | 4 |
| Comp. Ex.13 | Pt-SO₄²⁻/ZrO₂ | 51 | 90 |

| | | | |
|---|---|---|---|
| (a) conversion of pentane, mol% | | | |
| (b) selectivity to isopentane, mol% | | | |

## Claims

1. A solid acid catalyst for paraffin conversion which comprises an acidic salt of a heteropoly acid wherein a portion of the hydrogen ions in the heteropoly acid has been replaced by other cations selected from alkali metal ions, alkaline earth metal ions and ammonium ions, and a metal of Group VIII having a content of 0.05% to 30% by weight, based on the total amount of the catalyst.

2. A solid acid catalyst according to claim 1, wherein the metal of Group VIII is a metal selected from Ru, Rh, Pd, Os, Ir and Pt.

3. A solid acid catalyst according to claim 1, wherein the heteropoly acid is represented by the following general formula(1):
H₍₈₀₋ₘ₋₁₂ₙ₎[X^{m+}Yⁿ⁺₁₂O₄₀] (1)
wherein X represents a hetero-atom selected from P, As, Si and Ge; Y represents a poly-atom which is at least one transition metal selected from Mo, W, Nb and V; m and n represent the valence number of X and Y, respectively.

4. A solid acid catalyst according to claim 1, wherein the acidic salt of the heteropoly acid is represented by the following general formula (2):
L^{ℓ+}ₖH_{(80-m-12n-k·ℓ)}[X^{m+}Yⁿ⁺₁₂O₄₀] (2)
wherein L is one ion selected from alkali metal ions, alkaline earth metal ions and ammonium ions; X represents a hetero-atom selected from P, As, Si and Ge; Y represents a poly-atom which is at least one transition metal from Mo, W, Nb and V; ℓ, m and n represent the valence number of L, X and Y, respectively; k represents the number of L and a number of over 0 and below (80-m-12n)/ℓ.

5. A solid acid catalyst according to claim 4, wherein X is P or Si and Y is W or Mo.

6. A solid acid catalyst according to claim 1, wherein the acidic salt of the heteropoly acid is represented by the following general formula (3):
AₓH₍₃₋ₓ₎PY₁₂O₄₀ (3)
wherein A represents one ion selected from alkali metal ions and ammonium ion; Y represents at least one atom selected from W and Mo; x represents a number of 2.2 to 2.8.

7. A process for paraffin conversion which comprises the steps of contacting the paraffin in the presence of hydrogen with a solid acid catalyst comprising an acidic salt of a heteropoly acid wherein a portion of the hydrogen ions in the heteropoly acid has been replaced by other cations selected from alkali metal ions, alkaline earth metal ions and ammonium ions, and a metal of Group VIII having a content of 0.05% to 30% by weight, based on the total amount of the catalyst.

8. A process for paraffin conversion according to claim 7, wherein the metal of Group VIII is a metal selected from Ru, Rh, Pd, Os, Ir and Pt.

9. A process for paraffin conversion according to claim 7, wherein the heteropoly acid is represented by the following general formula (1):
H₍₈₀₋ₘ₋₁₂ₙ₎[X^{m+}Yⁿ⁺₁₂O₄₀] (1)
wherein X represents a hetero-atom selected from P, As, Si and Ge; Y represents a poly-atom which is at least one transition metal selected from Mo, W, Nb and V; m and n represent the valence number of X and Y, respectively.

10. A process for paraffin conversion according to claim 7, wherein the acidic salt of the heteropoly acid is represented by the following general formula (2):
L^{ℓ+}ₖH_{(80-m-12n-k·ℓ)}[X^{m+}Yⁿ⁺₁₂O₄₀] (2)
wherein L is one ion selected from alkali metal ions, alkaline earth metal ions and ammonium ions; X represents a hetero-atom selected from P, As, Si and Ge; Y represents a poly-atom which is at least one transition metal selected from Mo, W, Nb and V; ℓ, m and n represent the valence number of L, X and Y, respectively; k represents the number of L and a number of over 0 and below (80-m-12n)/ℓ.

11. A process for paraffin conversion according to claim 10, wherein X is P or Si and Y is W or Mo.

12. A process for paraffin conversion according to claim 7, wherein the acidic salt of the heteropoly acid is represented by the following general formula (3):
AₓH₍₃₋ₓ₎PY₁₂O₄₀ (3)
wherein A represents one ion selected from alkali metal ions and ammonium ions; Y represents at least one atom selected from W and Mo; x represents a number of 2.2 to 2.8.

13. A process for paraffin isomerization which comprises the steps of contacting the paraffin in the presence of hydrogen with a solid acid catalyst comprising an acidic salt of a heteropoly acid wherein a portion of the hydrogen ions in the heteropoly acid has been replaced by other cations selected from alkali metal ions, alkaline earth metal ions and ammonium ions, and a metal of Group VIII having a content of 0.05% to 30% by weight, based on the total amount of the catalyst, thereby carrying out paraffin isomerization.

## Patentansprüche

1. Fester saurer Katalysator zur Paraffinkonvertierung, der ein saures Salz einer Heteropolysäure, worin ein Anteil der Wasserstoffionen der Heteropolysäure durch andere Kationen, ausgewählt aus Alkalimetallionen, Erdalkalimetallionen und Ammoniumionen, ersetzt wurde, und ein Metall der Gruppe (VIII) mit einem Gehalt von 0,05 bis 30 Gew.%, bezogen auf die Gesamtmenge des Katalysators, umfasst.

2. Fester saurer Katalysator gemäss Anspruch 1, worin das Metall der Gruppe (VIII) ein aus Ru, Rh, Pd, Os, Ir und Pt ausgewähltes Metall ist.

3. Fester saurer Katalysator gemäss Anspruch 1, worin die Heteropolysäure durch die folgende allgemeine Formel (1) dargestellt wird:
H₍₈₀₋ₘ₋₁₂ₙ₎[X^{m+}Yⁿ⁺₁₂O₄₀] (1)
worin X ein Heteroatom, ausgewählt aus P, As, Si und Ge, darstellt; Y ein Polyatom darstellt, das wenigstens ein Übergangsmetall ist, ausgewählt aus Mo, W, Nb und V; und m und n die Wertigkeit von X bzw. Y darstellen.

4. Fester saurer Katalysator gemäss Anspruch 1, worin das saure Salz der Heteropolysäure durch die folgende allgemeine Formel (2) dargestellt wird:
L^{ℓ+}ₖH_{(80-m-12n-k·ℓ)}[X^{m+}Yⁿ⁺₁₂O₄₀] (2)
worin L ein Ion ist, ausgewählt aus Alkalimetallionen, Erdalkalimetallionen und Ammoniumionen; X ein Heteroatom darstellt, ausgewählt aus P, As, Si und Ge; Y ein Polyatom darstellt, das wenigstens ein Übergangsmetall aus Mo, W, Nb und V ist; ℓ, m und n die Wertigkeit von L, X bzw. Y darstellen; und k die Anzahl von L und eine Zahl über 0 und unter (80-m-12n)/ℓ darstellt.

5. Fester saurer Katalysator gemäss Anspruch 4, worin X P oder Si und Y W oder Mo ist.

6. Fester saurer Katalysator gemäss Anspruch 1, worin das saure Salz der Heteropolysäure durch die folgende allgemeine Formel (3) dargestellt wird:
AₓH₍₃₋ₓ₎PY₁₂O₄₀ (3)
worin A ein Ion darstellt, ausgewählt aus Alkalimetallionen und Ammoniumionen; Y wenigstens ein Atom darstellt, ausgewählt aus W und Mo; x eine Zahl von 2,2 bis 2,8 darstellt.

7. Verfahren zur Paraffinkonvertierung, das die folgenden Schritte umfasst: In-Kontakt-Bringen von Paraffin in Gegenwart von Wasserstoff mit einem festen sauren Katalysator, umfassend ein saures Salz einer Heteropolysäure, worin ein Teil der Wasserstoffionen in der Heteropolysäure durch andere Kationen, ausgewählt aus Alkalimetallionen, Erdalkalimetallionen und Ammoniumionen, ersetzt wurde, und ein Metall der Gruppe (VIII) mit einem Gehalt von 0,05 bis 30 Gew.%, bezogen auf die Gesamtmenge des Katalysators.

8. Verfahren zur Paraffinkonvertierung gemäss Anspruch 7, worin das Metall der Gruppe (VIII) ein aus Ru, Rh, Pd, Os, Ir und Pt ausgewähltes Metall ist.

9. Verfahren zur Paraffinkonvertierung gemäss Anspruch 7, worin die Heteropolysäure durch die folgende allgemeine Formel (1) dargestellt wird:
H₍₈₀₋ₘ₋₁₂ₙ₎[X^{m+}Yⁿ⁺₁₂O₄₀] (1)
worin X ein Heteroatom darstellt, ausgewählt aus P, As, Si und Ge; Y ein Polyatom darstellt, das wenigstens ein Übergangsmetall ist, ausgewählt aus Mo, W, Nb und V; und m und n die Wertigkeit von X bzw. Y darstellen.

10. Verfahren zur Paraffinkonvertierung gemäss Anspruch 7, worin das saure Salz der Heteropolysäure durch die folgende allgemeine Formel (2) dargestellt wird:
L^{ℓ+}ₖH_{(80-m-12n-k·ℓ)}[X^{m+}Yⁿ⁺₁₂O₄₀] (2)
worin L ein Ion ist, ausgewählt aus Alkalimetallionen, Erdalkalimetallionen und Ammoniumionen; X ein Heteroatom darstellt, ausgewählt aus P, As, Si und Ge; Y ein Polyatom darstellt, das wenigstens ein Übergangsmetall ist, ausgewählt aus Mo, W, Nb und V; ℓ, m und n die Wertigkeit von L, X bzw. Y darstellen; und k die Anzahl von L und eine Zahl über 0 und unter (80-m-12n)/ℓ darstellt.

11. Verfahren zur Paraffinkonvertierung gemäss Anspruch 10, worin X P oder Si und Y W oder Mo ist.

12. Verfahren zur Paraffinkonvertierung gemäss Anspruch 7, worin das saure Salz der Heteropolysäure durch die folgende allgemeine Formel (3) dargestellt wird:
AₓH₍₃₋ₓ₎PY₁₂O₄₀ (3)
worin A ein Ion darstellt, ausgewählt aus Alkalimetallionen und Ammoniumionen; Y wenigstens ein Atom darstellt, ausgewählt aus W und Mo; und x eine Zahl von 2,2 bis 2,8 darstellt.

13. Verfahren zur Paraffinisomerisierung, das die folgenden Schritte umfasst: In-Kontakt-Bringen von Paraffin in Gegenwart von Wasserstoff mit einem festen sauren Katalysator, umfassend ein saures Salz einer Heteropolysäure, worin ein Teil der Wasserstoffionen in der Heteropolysäure durch andere Kationen, ausgewählt aus Alkalimetallionen, Erdalkalimetallionen und Ammoniumionen, ersetzt wurde, und ein Metall der Gruppe (VIII) mit einem Gehalt von 0,05 bis 30 Gew.%, bezogen auf die Gesamtmenge des Katalysators, wodurch die Paraffinisomerisierung durchgeführt wird.

## Revendications

1. Catalyseur acide solide pour la conversion de paraffine, qui comporte un sel acide d'un hétéropolyacide, dans lequel une partie des ions hydrogène de l'hétéropolyacide a été remplacée par d'autres cations choisis parmi des ions de métaux alcalins, des ions de métaux alcalino-terreux et des ions ammonium, et un métal du groupe VIII à une teneur de 0,05% à 30% en poids, calculés sur la quantité totale de catalyseur.

2. Catalyseur acide solide selon la revendication 1, dans lequel le métal du groupe VIII est un métal choisi parmi Ru, Rh, Pd, Os, Ir et Pt.

3. Catalyseur acide solide selon la revendication 1, dans lequel l'hétéropolyacide est représenté par la formule générale (1) ci-dessous:
H₍₈₀₋ₘ₋₁₂ₙ₎[X^{m+}Yⁿ⁺₁₂O₄₀] (1)
dans laquelle X représente un hétéroatome choisi parmi P, As, Si et Ge; Y représente un polyatome qui est au moins un métal de transition choisi parmi Mo, W, Nb et V; m et n représentent les nombres de valence respectifs de X et Y.

4. Catalyseur acide solide selon la revendication 1, dans lequel le sel acide de l'hétéropolyacide est représenté par la formule générale (2) ci-dessous:
L^{ℓ+}ₖH_{(80-m-12n-k·ℓ)}[X^{m+}Yⁿ⁺₁₂O₄₀] (2)
dans laquelle L est un ion choisi parmi des ions de métaux alcalins, des ions de métaux alcalino-terreux et des ions ammonium; X représente un hétéroatome choisi parmi P, As, Si et Ge; Y représente un polyatome qui est au moins un métal de transition choisi par Mo, W, Nb et V; ℓ, m et n représentent les nombres de valence respectifs de L, X et Y; k représente le nombre de L et est un nombre compris entre 0 et (80-m-12n)/ℓ).

5. Catalyseur acide solide selon la revendication 4, dans lequel X représente P ou Si, et Y représente W ou Mo.

6. Catalyseur acide solide selon la revendication 1, dans lequel le sel acide de l'hétéropolyacide est représenté par la formule générale (3) ci-dessous:
AₓH₍₃₋ₓ₎PY₁₂O₄₀ (3)
dans laquelle A représente un ion choisi parmi des ions de métaux alcalins et l'ion ammonium; Y représente au moins un atome choisi parmi W et Mo; x représente un nombre compris entre 2,2 et 2,8.

7. Procédé pour la conversion de paraffine, qui comporte les étapes consistant à, en présence d'hydrogène, mettre la paraffine en contact avec un catalyseur acide solide comportant un sel acide d'un hétéropolyacide dans lequel une partie des ions hydrogène de l'hétéropolyacide a été remplacée par d'autres cations choisis parmi des ions de métaux alcalins, des ions de métaux alcalino-terreux et des ions ammonium, et un métal du groupe VIII à une teneur de 0,05% à 30% en poids, calculés sur la quantité totale du catalyseur.

8. Procédé pour la conversion de paraffine selon la revendication 7, dans lequel le métal du groupe VIII est un métal choisi parmi Ru, Rh, Pd, Os, Ir et Pt.

9. Procédé pour la conversion de paraffine selon la revendication 7, dans lequel l'hétéropolyacide est représenté par la formule générale (1) ci-dessous:
H₍₈₀₋ₘ₋₁₂ₙ₎[X^{m+}Yⁿ⁺₁₂O₄₀] (1)
dans laquelle X représente un hétéroatome choisi parmi P, As, Si et Ge; Y représente un polyatome qui est au moins un métal de transition choisi parmi Mo, W, Nb et V; m et n représentent les nombres de valence respectifs de X et Y.

10. Procédé pour la conversion de paraffine selon la revendication 7, dans lequel le sel acide de l'hétéropolyacide est représenté par la formule générale (2) ci-dessous:
L^{ℓ+}ₖH_{(80-m-12n-k·ℓ)}[X^{m+}Yⁿ⁺₁₂O₄₀] (2)
dans laquelle L est un ion choisi parmi des ions de métaux alcalins, des ions de métaux alcalino-terreux et des ions ammonium; X représente un hétéroatome choisi parmi P, As, Si et Ge; Y représente un polyatome qui est au moins un métal de transition choisi par Mo, W, Nb et V; ℓ, m et n représentent les nombres de valence respectifs de L, X et Y; k représente le nombre de L et est un nombre compris entre 0 et (80-m-12n)/ℓ.

11. Procédé pour la conversion de paraffine selon la revendication 10, dans lequel X représente P ou Si et Y représente W ou Mo.

12. Procédé pour la conversion de paraffine selon la revendication 7, dans lequel le sel acide de l'hétéropolyacide est représenté par la formule générale (3) ci-dessous:
AₓH₍₃₋ₓ₎PY₁₂O₄₀ (3)
dans laquelle A représente un ion choisi parmi des ions de métaux alcalins et des ions ammonium; Y représente au moins un atome choisi parmi W et Mo; x représente un nombre compris entre 2,2 et 2,8.

13. Procédé pour l'isomérisation de paraffine, qui comporte les étapes consistant à mettre, en présence d'hydrogène, la paraffine en contact avec un catalyseur acide solide comportant un sel acide d'un hétéropolyacide dans lequel une partie des ions hydrogène de l'hétéropolyacide a été remplacée par d'autres cations choisis parmi des ions de métaux alcalins, des ions de métaux alcalino-terreux et des ions ammonium, et un métal du groupe VIII à une teneur de 0,05% à 30% en poids, calculés sur la quantité totale du catalyseur, pour ainsi réaliser l'isomérisation de la paraffine.
